# EUROPÄISCHE PATENTANMELDUNG

(11) **EP 1 702 594 A1**
(43) Veröffentlichungstag der Anmeldung: **20.09.2006**
(21) Anmeldenummer: 06005006.9
(22) Anmeldetag: 11.03.2006
(51) Int. Cl.: A61F 5/02

(54) **Korsett, insbesondere zur Skoliosebehandlung**

(30) Priorität: 18.03.2005 DE 102005012564
(71) Anmelder: Weiss, Hans-Rudolf, Dr. med., 55566 Bad Sobernheim (DE)
(72) Erfinder: Weiss, Hans-Rudolf, Dr. med., 55566 Bad Sobernheim (DE)
(74) Vertreter: Becker, Bernd

(57) **Zusammenfassung**

Ein Korsett, insbesondere zur Skoliosebehandlung, umfasst an einem vorderen und/oder hinteren Längsträger(1, 2) auswechselbar befestigte flächige Druckkörper (3), wobei Ausweichraum (4) für verdrängtes Rumpfvolumen vorgesehen ist. Die Druckkörper (3) und/oder die Längsträger (1, 2) sind nach einem Baukastenprinzip gestaltet und weisen unterschiedliche Geometrien und/oder Größen auf.

## Beschreibung

Die Erfindung bezieht sich auf ein Korsett, insbesondere zur Skoliosebehandlung, mit an einem vorderen und/oder hinteren Längsträger auswechselbar befestigten flächigen Druckkörpern, wobei Ausweichraum für verdrängtes Rumpfvolumen vorgesehen ist.

Zur Behandlung von Wirbelsäulenverbiegungen, insbesondere Skoliose, sind verschiedene Korsettypen entwickelt worden. Diese umfassen unter anderen die so genannten teilaktiven starren Korsette, beispielsweise das Cheneau-, CBW-, Boston-, Stagnara-, Milwaukee-Korsett, die jeweils einerseits Druckzonen und andererseits Freiräume aufweisen. Auf vorstehende Bereiche, wie Rippenbuckel, wird Druck ausgeübt und gegenüberliegend ist Freiraum vorgesehen, damit die fortgedrückten Bereiche dorthin ausweichen. Die meisten vorhandenen Rumpforthesen wirken über seitliche Kräfte und wirken dabei mehr oder weniger auch auf den Rippenbuckel und/oder den Lendenwulst eine Derotationskraft (Entdrehkraft) aus. Lediglich beispielhaft werden aufgeführt ein Korsett nach der DE 29 21 015 C2 zur aktiven Korrektur der Wirbelsäule mit fortschreitender Regulierung und eine Variante eines Cheneau-Korsetts nach der DE 36 22 265 Al mit abnehmbarem Halsteil, das zur Therapie von hochthorakalen und cervikalen Verkrümmungen und bei Überhang dient.

Wichtig ist indessen die Behandlung nicht nur der Seitverbiegung, sondern auch die Berücksichtigung der sagittalen Verbiegung, was erst in neuerer Zeit berücksichtigt wird. Die nicht korrigierten Wirbelsäulenverbiegungen können langfristig zu Beeinträchtigungen der Funktion und zu Schmerzen führen. Da vielfach eine fast ganztägige Tragezeit des Korsetts über etliche Jahre benötigt wird, um langfristig eine Korrektur zu erzielen, sollte ein Korsett möglichst bequem zu tragen sein. Weiter mitbestimmend für die Akzeptanz des Korsetts und damit auch für den Therapieerfolg ist dessen Unauffälligkeit beim Tragen.

Mit dem so genannten Rigo-System und dem Cheneau-Korsett soll zusätzlich zur Korrektur der Seitverbiegung und Verdrehung der Wirbelsäule, insbesondere im Brustwirbelsäulenbereich, die Rückrundung der Brustwirbelsäule wieder hergestellt werden.

Als nachteilig hat sich erwiesen, dass sämtliche Korsette zu jeweils einem Termin ausgemessen werden bzw. eine Abdrucknahme, beispielsweise durch Gipsen, erfolgt, anschließend anprobiert und mit Hilfe von Röntgenaufnahmen kontrolliert sowie gegebenenfalls nachgebessert werden.

Anschließend erfolgen Routinekontrollen in 3 bis 6 monatigen Abständen. Bei der CAD basierten musterspezifischen Chêneau Bauweise werden im Hauptwachstumsschub mitunter bereits nach drei Monaten wachstumsbedingt Neuversorgungen notwendig. Dies kann bei der Stagnara Bauweise durch einfaches Verlagern und Weiten einzelner Redressionsspangen vermieden werden. Allerdings ist diese Versorgung nicht mustergenau. Es wird in der Regel lediglich Symmetrie angestrebt.

Der Erfindung liegt die Aufgabe zugrunde, ein Korsett zur Behandlung von Wirbelsäulendeformitäten, insbesondere zur Skoliosebehandlung, zu schaffen, das eine wirksame Korrektur erzielt, günstige Trageeigenschaften aufweist und dabei kostengünstig anzufertigen ist.

Erfindungsgemäß wird die Aufgabe dadurch gelöst, dass die Druckkörper und/oder die Längsträger nach einem Baukastenprinzip gestaltet sind und unterschiedliche Geometrien und/oder Größen aufweisen.

Die vorgefertigten Druckkörper werden in Abhängigkeit von einem individuellen Krümmungsmuster des Trägers des Korsetts, also einem Patienten, ausgewählt und an den beiden Längsträgern befestigt. Damit ist es nicht erforderlich, ein komplettes musterspezifisches Korsett neu zu fertigen, was mit einer Kostenreduzierung verbunden ist. Darüber hinaus entfallen aufgrund der schnellen Anfertigung des Korsetts in modularer Bauweise auch lange Wartezeiten zwischen dem Ausmessen bzw. der Abdrucknahme und der anschließenden Anprobe, die durch die komplette Neuanfertigung eines Korsetts bedingt sind. Während der Wartezeiten vor der Versorgung des Trägers des Korsetts, die in der Praxis bis zu acht Wochen betragen, beginnt die Passgenauigkeit des ausgemessenen Korsetts oftmals bereits zu leiden und eine Krümmungszunahme des Trägers des Korsetts kann dann manchmal trotz einer Behandlung mit einem Korsett nicht verhindert werden.

Der Vorteil der Erfindung liegt darin, dass sämtliche Bauteile des Korsetts in standardisierten Geometrien (Grundmustern) und Größen, die von einem erfahrenen Fachmann zu ermitteln bzw. in einschlägiger Fachliteratur beschrieben sind, in einer großen Stückzahl vorgefertigt werden können. Bestimmte Grundmuster sind durch die langjährige Anwendung der so genannten Rigo-Klassifikation bekannt. Bei der Anpassung des Korsetts an den jeweiligen Träger wird unter den entsprechenden Bauteilen ausgewählt und das individuelle Korsett zusammengesetzt. Bei einer wachstums- oder krankheitsbedingten Veränderung, insbesondere einem Längen- oder Breitenwachstum, des Trägers des Korsetts kann eine erneute Anpassung durch den einfachen Austausch einzelner Druckkörper bzw. Längsträger erfolgen. Um beispielsweise den größten Anteil der idiopathischen Skoliosen, nämlich bei Mädchen zwischen 12 und 16 Jahren, behandeln zu können, werden mindestens zwei, bevorzugt drei verschiedene Größen benötigt. Das Korsett lehnt sich demnach an die Bauweise nach Stagnara an, die die Möglichkeit bietet, die Pelottenanordnung leicht zu modifizieren und das Korsett somit an evtl. Breiten- und Längenwachstum anzupassen. Neuversorgungen wären auf diese Weise über lange Zeitintervalle zu vermeiden und zwar ohne die Behandlungsspezifität zu verlieren.

Bevorzugt sind die Druckkörper in ihrer Lage einstellbar befestigt. Zweckmäßigerweise sind die Längsträger physiologisch gebogen. Hierdurch ist eine abwechselnde Anordnung von nach vorne und nach hinten gerichteter Kräfte zu erzielen, durch die sich auf die Wirbelsäule wirkende Hebelkräfte und Drehmomente ergeben. Die Biegung der Längsträger entspricht den physiologischen sagittalen Schwingungen.

Vorteilhafterweise ist einer der Druckkörper als Beckenfassung ausgeführt. Vorzugsweise ist die Beckenfassung symmetrisch oder asymmetrisch ausgebildet. Eine symmetrische Beckenfassung wird dann gewählt, wenn das Becken des Trägers bei einem gradem Abgang der Lendenwirbelsäule auf dem Kreuzbein (Krümmungsmuster nach Lehnert-Schroth 3 BN) neutral einzustellen ist. Bei einem schrägen Abgang der Lendenwirbelsäule auf dem Kreuzbein, im Sinne einer lumbalen Krümmung ohne Keilverformung des Bandscheibenraumes L4/L5 in frontaler Ebene, ist eine Beckenkippung zur thorakalen Konvexseite vorzusehen, wozu eine asymmetrische Beckenfassung verwendet wird.

Nach einer Weiterbildung ist einer der Druckkörper als Lumbalspange ausgebildet. Die Lumbalspange ist bei einer geraden Einstellung der Lendenwirbelsäule auf dem Kreuzbein mit Abkippung von L3 zur thorakalen Konkavseite oder höher als eine Anlehnungszone im Sinne einer angedeuteten Dreieckspelotte vorzusehen (Rigo Non 3 non 4), die bei sekundärer lumbaler Krümmungsverstärkung aufgedoppelt werden kann. Bei einer Neigung von L4 zur thorakalen konvexen Seite, entspricht King - Typ IV, ist keine lumbale Druckzone oder Lumbalpelotte erforderlich. Liegt ein lumbaler Krümmungsscheitel bis L2 vor, ist eine lumbale Druckzone mit einer Dreieckspelotte unter Ausschluss der elften Rippe einzufügen, um einen Blockadeeffekt mit einem thorakalen Gegenhalt und eine mögliche Verstärkung der thorakalen Krümmung zu erzielen. Bei einem thorakolumbalem Krümmungsscheitel Th12/L1, vor allem bei Dekompensation des Lumbalbogens, erfolgt die Einbringung einer ausgedehnteren Lumbalpelotte unter Einschluss der zehnten und elften Rippe, da bei einem alleinigen Druck auf die elfte Rippe regelmäßig Schmerzsyndrome entstehen.

Vorzugsweise ist einer der Druckkörper als Thorakalspange ausgeführt. Bei einem rein lumbalem Krümmungsmuster sollte im vorderen Rippenbogenbereich zumindest die Andeutung eines Punktes 4 (nach Chêneau) und eines Freiraumes ventral vor der Konvexität des thorakalen Ausgleichsbogens eingebaut sein. Bei rein thorakalen Krümmungen ohne wesentliche zervikothorakale oder lumbale Gegenkrümmung sollte die Ausrichtung der Druckpelotte, also der Thorakalspange, thorakal einen Winkel von 40° bilden, und die ventrale Druckpelotte im Bereich Punkt 4 (nach Chêneau) sollte bei einer Pelottenhöhe von nicht mehr als 3 cm kaudokranial in der frontalen Hauptebene bis leicht frontolateral ausgerichtet sein. Bei mehrbogigen Krümmungen, so genannten double major oder doppelthorakalen Skoliosemustern, aber auch bei deutlicher lumbaler Gegenkrümmung ist die Ausrichtung der thorakalen Pelotte auf 50 bis 60° zu bringen, also mit mehr lateraler Abstützung und die ventrale Druckpelotte im Bereich Punkt 4 (nach Chêneau) sollte bei einer Pelottenhöhe von nicht mehr als 3 cm kaudokranial in der frontalen Hauptebene bis leicht frontolateral ausgerichtet sein. Zur Aufspreizung des Rippenbuckels muss allerdings im Regelfall die vordere Axillarlinie druckfrei sein.

In weiterer Ausgestaltung ist einer der Druckkörper als ventrale Spange ausgebildet. Die ventrale Druckpelotte im Bereich Punkt 4 (nach Chêneau) sollte bei einer Pelottenhöhe von nicht mehr als 3 cm kaudokranial in der frontalen Hauptebene bis leicht frontolateral ausgerichtet sein.

Bevorzugt ist einer der Druckkörper als Axillarspange ausgebildet. Bei thorakalen Krümmungen ohne zervikothorakalen Gegenbogen dient die Axillarspange der Aufspreizung der thorakalen Konkavität, um den Ansatz der thorakalen Derotationskräfte zu verbessern. Aus diesem Grund ist axillar kein Druck auf die oberen Rippen erforderlich. Die Axillarpelotte muss jedoch soweit herangestellt werden, dass der thorakalkonkavseitige Arm sich nicht abhebelt. Bei einem thorakalem Krümmungsscheitel bis Th7 muss zusätzlich die Thorakalpelotte kranial soweit reduziert werden, dass ohne ventrale Anlage der Schultergürtel nach dorsal über das Hypomochlion der Thorakalpelotte am unteren Rippenbuckel kippen kann. Bei einem thorakalen Krümmungsscheitel höher als Th7 muss die Scheitelwirbelhöhe beachtet werden, weshalb in diesen Fällen mit mangelndem Rückhalt der thorakalkonvexseitigen Schulter eine Corakoidalpelotte notwendig werden kann. Bei doppelthorakalen Krümmungen (Kippung Th1 zur thorakalen Konvexseite (King - Typ V)), ist im thorakalkonkavseitigen Axillarbereich eine seitliche Druckzone mit Druckpelotte einzufügen, welche den Schultergürtel noch weiter zur thorakalen Konvexseite hin hyperkompensiert, damit bei Erhalt der Orthoptie ein halbaktives Aufrichten der zervikothorakalen Krümmung erfolgen kann. Hierzu ist es notwendig, die Axillarpelotte 2 cm unter der maximalen Axillarhöhe zu kappen, damit ein leichter Rekompensationsraum oberhalb dieser Pelotte noch möglich ist

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispieles unter Bezugnahme auf die zugehörige Zeichnung näher erläutert. Es zeigt:

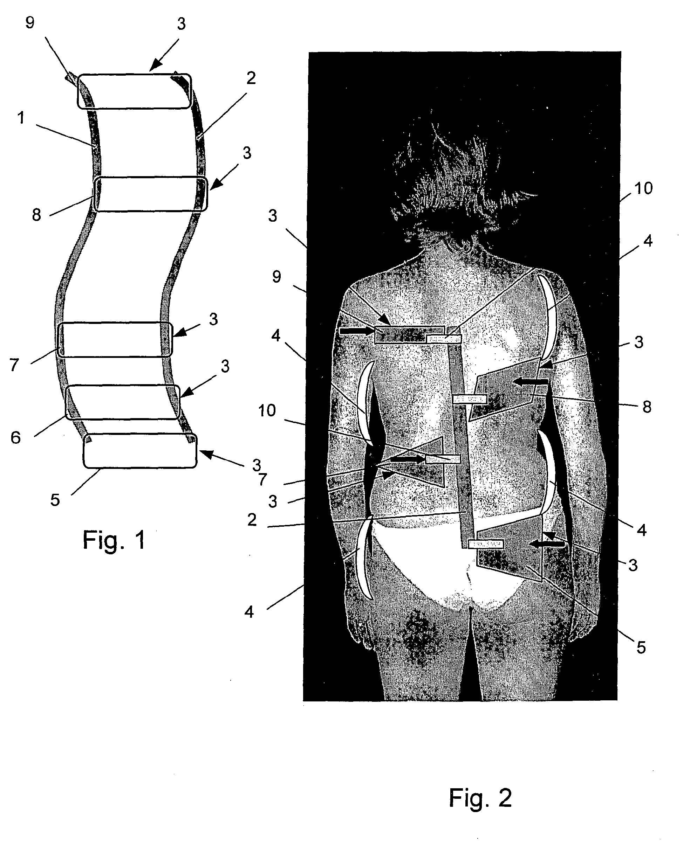

- Fig.1: eine schematische Seitendarstellung eines erfindungsgemäßen Korsetts und
- Fig.2: eine Darstellung des Korsetts nach Fig. 1 an einer Trägerin von hinten, in einer Variante einer Vielzahl musterspezifischer Konstruktionsmöglichkeiten.

Das Korsett umfasst einen vorderen Längsträger 1 und einen hinteren Längsträger 2, die jeweils entsprechend den physiologischen sagittalen Schwingungen vorgebogen sind, um eine abwechselnde Anordnung von nach vorne und nach hinten gerichteter auf die Wirbelsäule wirkender Kräfte für sagittale Korrekturen zu erzielen. An den Längsträgern 1, 2 sind flächige Druckkörper 3 über in sich starre Verbindungselemente 10 auswechselbar befestigt, von denen zumindest einzelne zum An- bzw. Ablegen des Korsetts von den Längsträgern 1, 2 zu lösen sind. Zwischen den Druckkörpern 3 ist Ausweichraum 4 für verdrängtes Rumpfvolumen vorgesehen. Bei den Druckkörpern 3 handelt es sich im Einzelnen um eine Beckenfassung 5, die vorliegend asymmetrisch ausgeführt ist, eine Lumbalspange 6, eine ventrale Spange 7, eine Thorakalspange 8 und eine Axillarspange 9.

Die Druckkörper 3 und die beiden Längsträger 1, 2 werden nach dem Baukastenprinzip in unterschiedlichen definierten Geometrien und Größen vorgefertigt und zu einem an den jeweiligen Träger angepassten Korsett zusammengesetzt.

## Patentansprüche

1. Korsett, insbesondere zur Skoliosebehandlung, mit an einem vorderen und/oder hinteren Längsträger(1, 2) auswechselbar befestigten flächigen Druckkörpern (3), wobei Ausweichraum (4) für verdrängtes Rumpfvolumen vorgesehen ist, **dadurch gekennzeichnet, dass** die Druckkörper (3) und/oder die Längsträger (1, 2) nach einem Baukastenprinzip gestaltet sind und unterschiedliche Geometrien und/oder Größen aufweisen.

2. Korsett nach Anspruch 1, **dadurch gekennzeichnet, dass** die Druckkörper (3) in ihrer Lage einstellbar befestigt sind.

3. Korsett nach Anspruch 1, **dadurch gekennzeichnet, dass** die Längsträger (1, 2) physiologisch gebogen sind.

4. Korsett nach Anspruch 1 oder 2, **dadurch gekennzeichnet, dass** einer der Druckkörper (3) als Beckenfassung (5) ausgeführt ist.

5. Korsett nach Anspruch 4, **dadurch gekennzeichnet, dass** die Beckenfassung (5) symmetrisch oder asymmetrisch ausgebildet ist.

6. Korsett nach Anspruch 1 oder 2, **dadurch gekennzeichnet, dass** einer der Druckkörper (3) als Lumbalspange (6) ausgebildet ist.

7. Korsett nach Anspruch 1 oder 2, **dadurch gekennzeichnet, dass** einer der Druckkörper (3) als Thorakalspange (8) ausgeführt ist.

8. Korsett nach Anspruch 1 oder 2, **dadurch gekennzeichnet, dass** einer der Druckkörper (3) als ventrale Spange (7) ausgebildet ist.

9. Korsett nach Anspruch 1 oder 2, **dadurch gekennzeichnet, dass** einer der Druckkörper (3) als Axillarspange (9) ausgebildet ist.
